# DEMANDE DE BREVET EUROPEEN

(11) **EP 1 121 949 A1**
(43) Date de publication de la demande: **08.08.2001**
(21) Numéro de dépôt: 99403282.9
(22) Date de dépôt: 24.12.1999
(51) Int. Cl.: A61M 5/315, A61D 7/00

(54) **Propulseur manuel à engrenage tubulaire mû d'une seule main**

(71) Demandeur: Simonian, Gilbert Michel, 92350 Le Plessis - Robinson (FR)
(72) Inventeur: Simonian, Gilbert Michel, 92350 Le Plessis-Robinson (FR); Segard, Jean-Vincent Blaise, 92170 Vanves (FR)

(57) **Abrégé**

L'invention concerne un mécanisme qui relaie la main de l'homme dans la pression exercée sur les poignées d'un pistolet injectant des liquides ou des gaz. Il augmente la distance de parcours de l'axe du piston dans le but d'augmenter le volume déliverable par la seule force d'une seule main. Elle est particulièrement adaptée au domaine vétérinaire. Constituée de:
une poignée avant (A) entourant les autres pièces et présentant un rétrécissement rainuré à l'avant; une poignée arrière (B) qui propulse l'engrenage tubulaire (C) et l'axe de piston (D). La partie tubulaire centrale de (B) sert d'axe de rotation pour (C), de plus, comporte des ouvertures rectilignes guidant les excroissances de l'axe de piston (D) s'insérant en son sein, tout en en empêchant la rotation. L'engrenage (C) est bloqué longitudinalement par (B) et présente un filetage externe de large pas qui s'enfiche dans les rainures de la partie avant de (A). La face interne de (C) présente des rainures dont le pas est de signe inverse à celui du filetage externe de (C). L'axe de piston (D) présente à l'arrière des excroissances perpendiculaires qui suivent les guides de (B) et passent dans les rainures internes de (C).

La pression manuelle enfonce la partie arrière (B,C,D) dans les guides de (A) qui imposent une rotation de (C). L'axe (D) tend à suivre le mouvement de (C) mais, bloqué en rotation par les ouvertures de (B), glisse longitudinalement. Le parcours relatif de (D) par rapport à (B) s'ajoute à celui de (B) dans (A).

## Description

La présente invention concerne un dispositif mécanique pour augmenter la distance linéaire de poussée manuelle lorsque cette poussée doit être effectuée d'une seule main. Par exemple dans les pistolets doseurs ou drogueurs utilisés couramment en médecine vétérinaire, la poussée directe de la poignée arrière par une main adulte ne peut pas s'effectuer sur plus de 7 cm environ en raison des limites de l'écartement de la main et cela dans des pressions raisonnables, ce qui implique pour le moment un volume critique proche de 300 cm³.

Cette invention consiste en un mécanisme radial, axé et solidaire à la partie arrière du système à faire évoluer. Celui-ci accompagne le mouvement alternatif de la poussée linéaire et transfère la poussée de la main vers l'axe central entraînant le piston (dans le cas du pistolet pour liquide). Ces deux poussées s'additionnent, imprimant un mouvement de plus grande amplitude au piston, allant jusqu'à doubler sa course et par voie de conséquence doubler le volume éjectable.

L'action mécanique est un ensemble emboîté de transferts de forces. La main produit une force de poussée linéaire qui impose une rotation à un engrenage axial, cette rotation imprime une poussée linéaire à l'axe piston.

Les dessins annexés illustrent l'invention :
- la figure 1 représente en coupe longitudinale, le dispositif de l'invention dans son ensemble,
- la figure 2 représente une vue de face, le dispositif de l'invention dans son ensemble,
- la figure 3 représente une vue en perspective de l'engrenage tubulaire (C) (dans lequel est pratiquée une coupe sur un coté pour voir l'intérieur),
- la figure 4 représente une vue en perspective de la pièce d'entraînement arrière (B),
- la figure 5 représente une vue en perspective de la pièce avant (A),
- la figure 6 représente une vue en perspective de l'axe de piston (D).

Détail des figures :

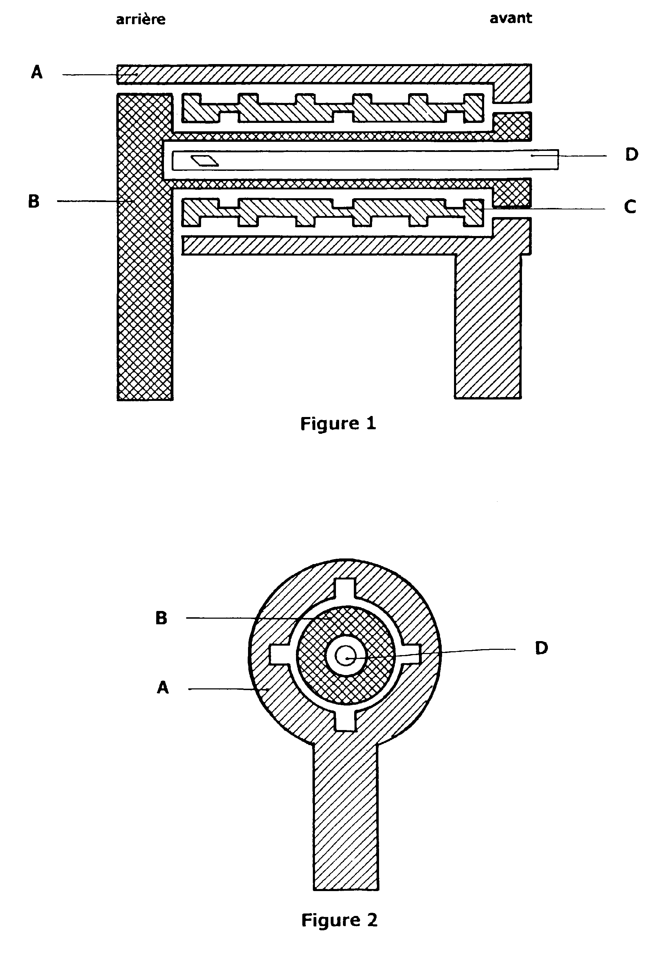

La figure 1 représente les quatre éléments nécessaires au fonctionnement de l'invention. Ils s'emboîtent de manière concentrique. (A) partie avant ou poignée avant est fixe, elle enveloppe l'ensemble du mécanisme de la poignée arrière (B, C, D). (B) est la poignée amère qui maintient l'engrenage longitudinal tubulaire (C) et l'axe de piston (D).

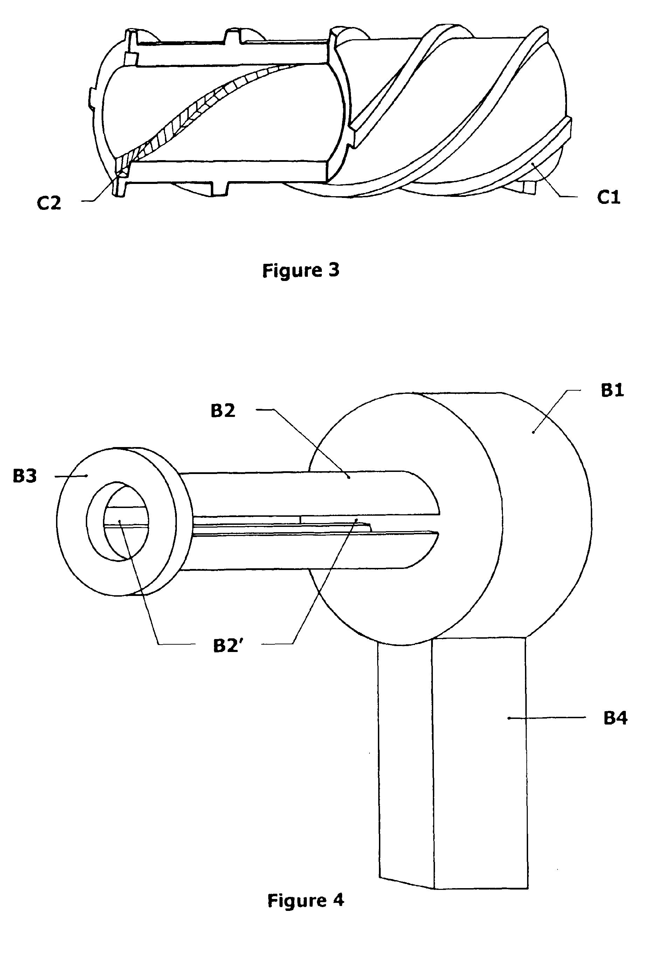

La figure 2 montre la forme concentrique de l'invention, (C) n'apparaît pas car il est en retrait des excroissances de (A) et (B) et perturberait la lisibilité de la figure.

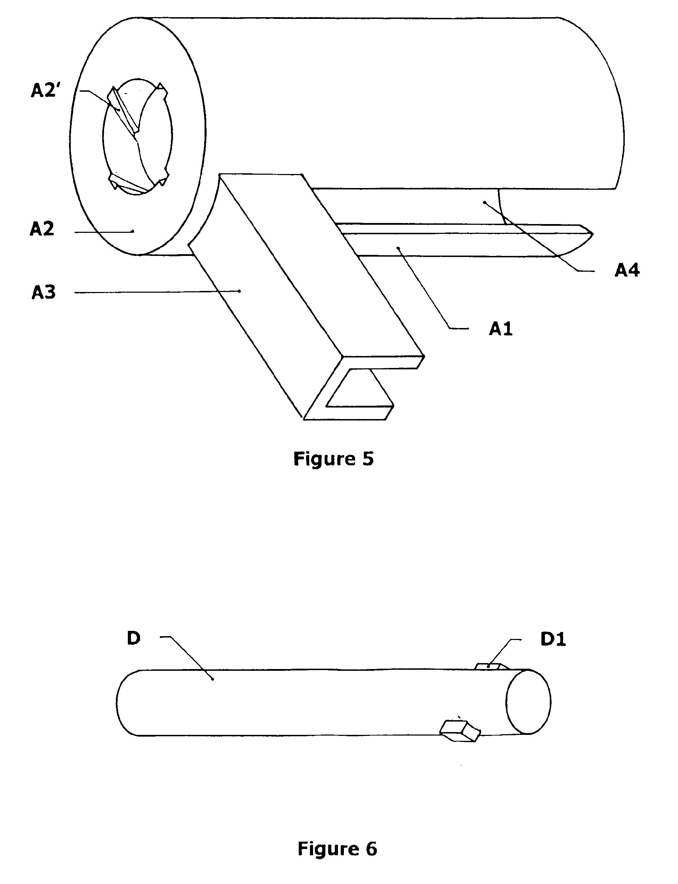

La poignée avant (A) détaillée dans la figure 5 est la plus externe. Elle se compose d'un tube (A1) dont l'extrémité avant comporte une excroissance rainurée (A2), d'une poignée (A3) et d'une saignée (A4) dans la partie arrière. Le diamètre général et la longueur du tube sont suffisants pour contenir les éléments de la partie arrière (B, C, D). L'excroissance à l'avant impose un passage et guide l'engrenage (C) par l'intermédiaire du filetage externe (Cl) de (C) dans ses rainures(A2'). La saignée (A4) permet le passage de la poignée arrière. La poignée (A3) est creuse et ouverte dans la face arrière pour permettre l'insertion de la poignée arrière en fin de course.

La partie arrière est composée d'une pièce d'entraînement (B) (détaillée dans la figure 4), d'un engrenage longitudinal tubulaire (C) (détaillé dans la figure 3) et d'un axe de piston (D) (détaillé dans la figure 6).

La partie (B) a trois caractéristiques distinctes. A l'arrière, un cylindre court (B1), d'un diamètre externe juste inférieur à celui de la face interne de (A1), il guide la partie arrière de l'invention dans le tube (A1) de (A) et reçoit la poussée de la main, par l'intermédiaire d'une poignée (B4). La partie centrale (B2) est un tube lisse de la longueur de (C) et comporte des ouvertures rectilignes (B2') qui permettent le passage pour les excroissances (Dl) de l'axe de piston (D) qui s'insère en son centre. Ces guides (B2') empêchent notamment la rotation de (D). Cette partie (B2) sert également d'axe de rotation pour (C). A l'extrémité avant, un tube (B3) (éventuellement amovible) dont le diamètre permet le passage par l'ouverture (A2) de (A) tout en bloquant les mouvements longitudinaux de (C) et retient (D) en fin de course au niveau des excroissances (D2).

L'engrenage longitudinal tubulaire (C) détaillé dans la figure 3 est le coeur de l'invention. Cette pièce mécanique permet le transfert des forces imprimées aux poignées vers l'axe de piston (D). Le tube qui compose (C) (dans l'exemple de réalisation de la figure 3) comporte un quadruple filetage externe (C1) de section rectangle dont la caractéristique est un angle de 45° par rapport à l'axe de symétrie de (C) et un double rainurage interne (C2) de section rectangle dont le pas est inversé (donc de - 45°). Le filetage externe (C1) correspond aux rainures (A2') de (A) et le filetage interne (C2) accepte les excroissances (Dl) de (D). Cette pièce peut uniquement jouer autour de son axe central dans une rotation complète sur la partie (B2) de la poignée arrière (B).

L'axe de piston (D) détaillé dans la figure 6 est la plus interne des pièces. Elle est composée d'un cylindre de diamètre correspondant à la face interne des parties tubulaires (B2) et (B3) de (B), et comporte des excroissances (Dl). Ces excroissances ont une forme de parallélogramme extrudé dont deux des quatre cotés sont parallèles à l'axe du cylindre (C) et aux ouvertures rectilignes (B2') de (B). Les autres cotés forment un angle de - 45° avec l'axe du cylindre, en regard des guides (C2) de (C). Inséré dans (B), l'axe de piston ne peut pas tourner sur lui même et sa liberté de mouvement longitudinal est bornée par la longueur des ouvertures rectilignes (B2') de (B) entre (B1) et (B3).

Le fonctionnement de ce mécanisme peut être analysé comme suit :
Une pression manuelle exercée sur la poignée arrière (B4) entraîne la partie arrière (B, C, D) dans (A), à travers la saignée (A4). Le filetage (C1) de l'engrenage (C) pénètre dans les rainures (A2') à l'avant de (A). Ce passage guide la pièce (C) et l'oblige à effectuer une rotation dans le sens de l'angle de filetage de (C1) afin de maintenir les filets (C1) en regard des rainures (A2) tout au long de la trajectoire longitudinale. Lorsque la pièce (C) tourne, ses rainures internes (C2) tendent a entraîné les excroissances (D1) qui sont insérées dans ce rainurage. Le rôle des ouvertures rectilignes (B2') est d'empêcher la rotation de (D), ne lui laissant qu'un mouvement alternatif pour seule liberté. Au cours de sa rotation, le tube (C) présente une partie différente de ses rainures (C2) devant les lignes que forment les ouvertures rectilignes (B2'). La force de rotation transmise par (C2) est alors convertie en mouvement alternatif car les extrémités des excroissances (Dl) doivent suivre les rainures (C2) en glissant sur les rebords de (C2) et ceux de (B2'). Cela ce traduit par un mouvement relatif à (D) par rapport à (B) qui s'ajoute au déplacement de l'ensemble de la partie arrière (B, C, D) lui-même relatif à la partie (A). Si l'extrémité avant de l'axe de piston (D) symbolise un piston, celui-ci parcourt une distance supérieure à celle parcourue par la main (ou par (B3)). En fin de course, les deux poignées se chevauchent afin d'optimiser la distance de parcours totale de la poignée arrière.

Les applications et les variantes de ce système sont multiples, elles ont pour but de pallier les limitations de la main humaine ou la difficulté d'utilisation structurelle de mécanismes à cuve . Dans le domaine particulier de l'instrumentation vétérinaire, le volume nécessaire à un traitement est souvent supérieur à la capacité des appareils à poussée directe. Ce mécanisme peut permettre d'augmenter la longueur de la cuve sans toucher au diamètre du piston, afin de délivrer plus de liquide tout en conservant une pression adéquate.

Cette invention pourra être utilisée dans des instruments délivrant des liquides manuellement et nécessitant une cuve d'un diamètre faible afin de passer par un orifice sans changer la contenance ; Pour des dispositifs servant à vider manuellement des réservoirs ou pour écoper.

Selon des modes particuliers de réalisation, le mécanisme peut avoir une ou plusieurs des caractéristiques suivantes et selon les appellations des figures 1 à 6:
a) le dosage des volumes peut s'effectuer à l'aide d'un écrou évoluant sur un filetage externe de la partie arrière de (A1) ;
b) des saillies dans la face interne de la partie (A1) guident et maintiennent dans l'axe de la cuve la partie (B1) rainurée en regard ;
c) un ressort entre les deux poignées permet le réamorçage et le remplissage automatique de la cuve (gaz ou liquide), il peut se trouver autour de l'engrenage (C) ou sous (A1)
d) le nombre d'excroissances (Dl) à l'arrière de l'axe de piston (D) peut varier de un à vingt, impliquant la réalisation d'un même nombre de saignées (B2') dans le tube (B2) ainsi que d'un même nombre de rainures (C2) dans la face interne de l'engrenage tubulaire (C),
e) le nombre de filetages (Cl) dans la face externe de l'engrenage tubulaire (C) peut varier de un à vingt, impliquant la réalisation du même nombre de saignées dans la partie (A2) à l'avant de la poignée avant (A),
f) les angles des filetages et rainures de la pièce (C) peuvent varier ensemble ou séparément en valeur absolue de manière à changer le coefficient multiplicateur de l'engrenage,
g) la partie (B3), à l'avant de la poignée arrière (B) peut être une pièce séparée et venir se visser sur un filetage placé à l'extrémité avant de (B2),
h) dans le cas g), une bague de maintien placée entre (B2) et le « bouchon » (B3) amovible, s'insérant entre les ouvertures (B2') permet le renfort de la pièce (B),
i) la « poignée avant » (A3) peut être montée sur un axe perpendiculaire à la pièce (A) et ainsi faciliter la saisie de l'appareil,

## Revendications

1. Dispositif pour augmenter la course linéaire d'un axe de piston (D) mû par une force d'une seule main. Caractérisé par un mécanisme radial (B, C, D) induisant la rotation d'un engrenage tubulaire (C) par les guides à l'avant d'une poignée avant (A) sous la force manuelle imprimée sur les poignées avant et arrière (A et B). Cette rotation l'engrenage (C) produit une poussée longitudinale sur l'axe de piston (D) par l'asservissement de l'axe de piston(D) par la poignée arrière (B).

2. Dispositif selon la revendication 1 caractérisé en ce que la pièce principale (C), intermédiaire entre la poussée manuelle et l'axe de piston, est un engrenage tubulaire dont les pas de vis interne et externes sont en angles inversés et les angles formés avec l'axe sont proches de 45°. Ces angles peuvent être différents ou identiques entre eux en valeur absolue afin de faire varier le coefficient multiplicateur provenant de l'engrenage.

3. Dispositif selon la revendication précédente caractérisé en ce que les sections des rainures et des filets de l'engrenage tubulaire (C) et du rétrécissement de la poignée avant, sont rectangles (en U).

4. Dispositif selon la revendication précédente caractérisé en ce que le nombre de filets et le nombre de rainures de l'engrenage tubulaire (C) peuvent varier indépendamment de 1 à 20, impliquant une variation en regard pour les éléments qui interagissent avec (C).

5. Dispositif selon la revendication précédente caractérisé en ce que les excroissances entraînant l'axe de piston (D) sont des parallélogrammes extrudés.

6. Dispositif selon la revendication précédente caractérisé en ce que les poignées s'imbriquent en fin de course pour minimiser la perte de distance due à l'épaisseur des poignées.

7. Dispositif selon la revendication précédente caractérisé en ce que pour automatiser le fonctionnement du dispositif, un ressort peut se placer entre les deux poignées, sous le corps de l'appareil ou autour de l'engrenage tubulaire (C).

8. Dispositif selon la revendication précédente caractérisé en ce que le dosage des volumes éjectables s'effectue par un contrôle de la distance de parcours grâce à un écrou se vissant sur le corps de la poignée avant (A) pour bloquer le recul de la poignée arrière (B).

9. Application d'un dispositif pour augmenter la course linéaire d'un axe de piston selon l'une quelconque des revendications précédentes à des pistolets drogueurs ou à des pistolets doseurs en usage dans le domaine vétérinaire.
